# EUROPÄISCHE PATENTSCHRIFT

(11) **EP 0 556 245 B1**
(45) Veröffentlichungstag und Bekanntmachung des Hinweises auf die Patenterteilung: **25.01.1995**
(21) Anmeldenummer: 91919525.5
(22) Anmeldetag: 06.11.1991
(51) Int. Cl.: A61K 31/425

(54) **VERWENDUNG VON THIAZOLOISOINDOLINON-DERIVATEN ALS ANTIVIRALE ARZNEIMITTEL**
USE OF THIAZOLO-ISOINDOLINONE DERIVATIVES AS ANTIVIRAL MEDICAMENTS
UTILISATION DE DERIVES DE LA THIAZOLOISOINDOLINONE COMME MEDICAMENTS ANTIVIRAUX

(30) Priorität: 10.11.1990 DE 4035809
(43) Veröffentlichungstag der Anmeldung: 25.08.1993
(73) Patentinhaber: BOEHRINGER MANNHEIM GMBH, 68298 Mannheim (DE)
(72) Erfinder: ZILCH, Harald, D-6800 Mannheim 31 (DE); MERTENS, Alfred, D-6905 Schriesheim (DE); LEINERT, Herbert, D-6148 Heppenheim (DE); LESER, Ulrike, D-8000 München 70 (DE); KÖNIG, Bernhard, D-8137 Berg 3 (DE); SEIDEL, Hans, D-8132 Tutzing (DE)
(74) Vertreter: Köster, Reinhold, Dr.
(86) Internationale Anmeldenummer: EP9102101
(87) Internationale Veröffentlichungsnummer: WO9208457

(56) Entgegenhaltungen:
- US-A- 3 334 113
- Journal of Medicinal Chemistry, vol., 21, no., 1., 1978, American Chem. Soc.; M. R. Hardnen et al.: "Thiazolinone analogues of indolmycin with antiviral and antibacterial activity", pages 82-87, see page 84, table 1, pages 84, 85, para.: Structure activity relationships

## Beschreibung

Gegenstand der vorliegenden Erfindung ist die neue Verwendung von Thiazoloisoindolinon-Derivaten zur Herstellung von Arzneimitteln mit antiviraler Wirkung.

Die vorliegende Erfindung betrifft die Verwendung von Verbindungen der allgemeinen Formel I 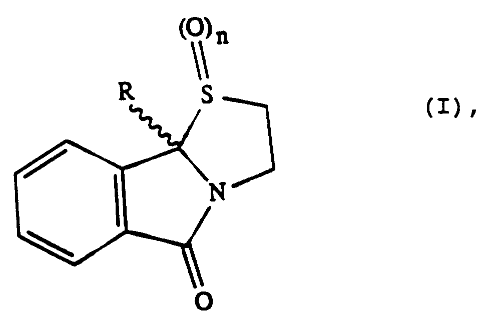
zur Herstellung von Arzneimitteln zur Behandlung von viralen oder retroviralen Infektionen, wobei
- R: ein Wasserstoffatom oder einen geradkettigen oder verzweigten, gesättigten oder ungesättigten aliphatischen Rest mit 1-7 Kohlenstoffatomen, der gegebenenfalls durch Phenyl substituiert sein kann, oder einen Phenylring, der gegebenenfalls ein- oder mehrfach durch C₁-C₄-Alkyl, C₁-C₄-Alkoxy, Hydroxy, Trifluormethyl, C₁-C₄-Alkylsulfonyl oder Halogen, wie Fluor, Chlor oder Brom, substituiert sein kann, bedeutet,
- n: für die Zahlen 0, 1 oder 2 steht,
sowie deren pharmakologisch verträgliche Salze und Tautomere.

Im US-Patent 3,334,113 ist u.a. das 9b-Phenyl-2,3-dihydrothiazolo-[2,3-a]isoindol-5(9bH)-on als entzündungshemmendes und anticonvulsives Arzneimittel beschrieben. Weitere Derivate des Thiazoloisoindolinons mit ähnlicher Wirkung und geringer Toxizität sind aus der schweizerischen Patentanmeldung CH-469,733 (R=Alkyl, n=0) und der belgischen Patentanmeldung 659,528 bzw. dem US-Patent 3,646,022 bekannt.

In U.S. 2,860,985 und der belgischen Patentanmeldung 564,592 werden Thiazoloisoindolinone zur Stabilisierung und als Kontrastmittel für photographische Emulsionen verwendet.

Die Synthese der Verbindungen ist außer in den genannten Patentanmeldungen auch in J. Org. Chem. 30, 1506 (1965) sowie J. Org. Chem. 34, 165 (1969) beschrieben.

Es wurde nun überraschenderweise gefunden, daß diese Verbindungen eine ausgeprägte antivirale Wirkung aufweisen, und sich daher besonders gut zur Behandlung von viralen bzw. retroviralen Infektionen eignen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrom (AIDS), den AIDS-related-complex (ARC) und deren Vorstadien, Herpes-, Cytomegalie-Virus (CMV)-, Influenza-und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir^{R}, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431). Die Verbindungen der allgemeinen Formel I besitzen diese Nachteile nicht. Sie wirken antiviral, ohne in pharmakologisch relevanten Dosen cytotoxisch zu sein.

Die Verbindungen der vorliegenden Erfindung weisen wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papilloma-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und -2, verursacht werden.

Besonders geeignet erscheinen die Verbindungen der Formel I zur Behandlung der klinischen Manifestationen der retroviralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Es konnte nun nachgewiesen werden, daß Verbindungen der allgemeinen Formel I die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw. Nature 325, 773 1987).

Da ein sehr großer Bedarf an Chemotherapeutica besteht, die möglichst spezifisch mit retroviral bedingten Erkrankungen oder deren Symptomen interferieren, ohne die normal ablaufenden natürlichen Körperfunktionen zu beeinflussen, könnten die genannten Verbindungen vorteilhaft prophylaktisch oder therapeutisch bei der Behandlung von Krankheiten eingesetzt werden, bei denen eine retrovirale Infektion von pathophysiologischer, symptomatischer oder klinischer Relevanz ist.

In der Formel I bedeutet R einen geradkettigen oder verzweigten, gesättigten oder ungesättigten aliphatischen Rest, insbesondere einen Alkylrest, mit 1-7, vorzugsweise 1-4 Kohlenstoffatomen, wie z. B. Methyl, Ethyl oder Isopropyl. Der aliphatische Rest kann auch durch eine Phenylgruppe substituiert sein, so daß R einen Phenylalkylrest, wie z. B. Benzyl, darstellt. Als ungesättigte Reste kommen C₂-C₇-Alkenyl- oder C₂-C₇-Alkinylgruppen in Frage. R kann ferner einen Phenylring darstellen, der unsubstituiert oder ein- oder mehrfach substituiert sein kann. Bevorzugt ist der Phenylring ein- oder zweifach substituiert. Als Substituenten kommen beispielsweise die Methyl-, Ethyl-, Methoxy- oder Ethoxygruppe in Frage.

Bevorzugt bedeutet R eine Phenylgruppe, die ein- bis dreifach substituiert sein kann durch die folgenden Reste: C₁-C₄-Alkyl, insbesondere Methyl, Ethyl, n-Propyl oder i-Propyl; Halogen, insbesondere Fluor, Chlor oder Brom; Trifluormethyl, Hydroxy, C₁-C₄-Alkoxy, insbesondere Methoxy oder Ethoxy. Besonders bevorzugt sind solche Derivate, die in 3- oder 4-Stellung des Phenylrings einen Substituenten tragen. Disubstituierte Phenylreste sind vorzugsweise die in 3,5-, 3,4-, 2,4- oder 2,5-Stellung substituierten Derivate.

Die Arzneimittel enthaltend mindestens eine Verbindung der Formel I zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragées, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäß verwendeten Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Die erfindungsgemäß verwendeten Verbindungen der allgemeinen Formel I können nach Vorschriften der im Stand der Technik genannten Patentanmeldungen bzw. Literaturstellen hergestellt werden.

Im Sinne der vorliegenden Erfindung kommen außer den in den Beispielen genannten Verbindungen und der durch Kombination aller in den Ansprüchen genannten Bedeutungen der Sustituenten die folgenden Verbindungen der Formel I in Frage:
1. 9b-(2,4-Dimethylphenyl)-2,3-dihydrothiazolo-[2,3-a]isoindol-5(9bH)-on
2. 9b-(4-Trifluormethylphenyl)-2,3-dihydrothiazolo-[2,3-a]isoindol-5(9bH)-on
3. 9b-(4-Ethoxyphenyl)-2,3-dihydrothiazolo-[2,3-a]isoindol-5-(9bH)-on
4. 9b-(3-Fluorphenyl)-2,3-dihydrothiazolo-[2,3-a]isoindol-5-(9bH)-on
5. 9b-(4-Methylsulfonylphenyl)-2,3-dihydrothiazolo-[2,3-a]isoindol-5(9bH)-on
6. 9b-Phenyl-2,3-dihydrothiazolo-[2,3-a]isoindol-5(9bH)-on-1-oxid

### Beispiel 1

### Hemmung der Reversen Transkriptase (RT) durch 9b-Phenyl-2,3-dihydro-thiazolo-[2,3-a]isoindol-5(9bH)-on

Das Screeningtestsystem beinhaltet die gereinigte RT aus HIV-1, die durch gentechnologische Methoden in E. coli exprimiert wurde, sowie die Komponenten des Initiationskomplexes, wie die in-vitro-Transkripte des HIV-LTR's mit der benachbarten Primer Binding Site als Template und einem zur Primer Binding Site komplementären 18mer Oligonukleotid als Primer. Gemessen wurde der [³H]-Thymidin-5'-triphosphat-Einbau durch Auszählen im β-Counter.

### Ergebnisse:

| Substanz | Hemmung der HIV-RT IC₅₀[M] |
|---|---|
| 3'-Azido-3'-desoxythymidin-5'-triphosphat [AZT-TP] | 6.0 x 10⁻⁶ |
| 3.2 | 4.0 x 10⁻⁶ |
| 3.4 | 4.0 x 10⁻⁶ |
| 3.8 | 0.8 x 10⁻⁶ |
| 3.12 | 1.1 x 10⁻⁶ |
| 3.15 | 1.0 x 10⁻⁶ |
| 3.18 | 0.95 x 10⁻⁶ |
| 3.30 | 0.7 x 10⁻⁶ |
| 4 | 1.4 x 10⁻⁶ |

### Beispiel 2

2.3-Dihydrothiazolo-[2.3-a]isoindol-5(9bH)-on wurde analog zu J. Am. Chem. Soc. 80, 702 (1958) in 64 % Ausbeute hergestellt und aus Ethanol umkristallisiert.
Schmp. 101-102°C (Lit.: 97-100°C).

### Beispiel 3

### 9b-(3,5-Dimethylphenyl)-2,3-dihydrothiazolo-[2,3-a]isoindol-5(9bH)-on

10 mmol 2-(3,5-Dimethylbenzoyl)benzoesäure wurden in 100 ml Xylol gelöst und nach Zugabe von 20 mmol Cysteamin sowie einer katalytischen Menge p-Toluolsulfonsäure 2 h am Wasserabscheider unter Rückfluß erhitzt. Dann wurde das Lösungsmittel im Vakuum entfernt und der Rückstand aus Ethanol umkristallisiert. Ausbeute 53 %, Schmp. 163°C.

Analog zu Beispiel 3 wurden folgende Verbindungen hergestellt:

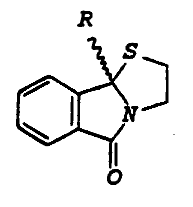

| Bsp. | R | Ausb. | Schmp.[°C] |
|---|---|---|---|
| 3.1 | 4-Methoxyphenyl | 31 % | öl |
| 3.2 | 4-Chlorphenyl | 47 % | 127-131 |
| 3.3 | 4-Methylphenyl | 81 % | 72-75 |
| 3.4 | 3,4-Dichlorphenyl | 60 % | öl |
| 3.5 | 3,4-Dimethylphenyl | 51 % | 155-157 |
| 3.6 | 4-Isopropylphenyl | 30 % | öl |
| 3.7 | 3-Methylphenyl | 78 % | 120-122 |
| 3.8 | 2,3-Dimethylphenyl | 74 % | 191-195 |
| 3.9 | 3-Isopropylphenyl | 42 % | 99-101 |
| 3.10 | 3-Ethylphenyl | 27 % | öl |
| 3.11 | 3-Chlorphenyl | 49 % | 132-134 |
| 3.12 | 3-Methoxyphenyl | 49 % | 141 |
| 3.13 | 3,5-Dichlorphenyl | 77 % | 108 |
| 3.14 | 4-Fluorphenyl | 56 % | 68 |
| 3.15 | 3-Trifluormethylphenyl | 40 % | 105 |
| 3.16 | 2-Hydroxy-4-methoxyphenyl | 37 % | 176 |
| 3.17 | 4-Hydroxyphenyl | 43 % | 240 |
| 3.18 | 2,5-Dimethylphenyl | 74 % | 91 |
| 3.19 | 4-Bromphenyl | 66 % | 145-147 |
| 3.20 | 3-Hydroxyphenyl | 45 % | 138-140 |
| 3.21 | 4-Ethylphenyl | 71 % | öl |
| 3.22 | 2-Chlorphenyl | 49 % | 150 |
| 3.23 | 2-Methylphenyl | 37 % | 110 |
| 3.24 | Benzyl | 18 % | 106-110 |
| 3.25 | Pentyl | 72 % | öl |
| 3.26 | Butyl | 61 % | öl |
| 3.27 | Methyl | 48 % | öl |
| 3.28 | Propyl | 77 % | 78-83 |
| 3.29 | Hexyl | 67 % | öl |
| 3.30 | Phenyl | 91 % | 108-110 |

### Beispiel 4

### 9b-Phenyl-2,3-dihydrothiazolo-[2,3-a]isoindol-5(9bH)-on-1,1-dioxid

1.3 g 9b-Phenyl-2,3-dihydrothiazolo-[2,3-a]isoindol-5(9bH)-on wurden in 12.5 ml Dichlormethan gelöst und mit einer Lösung aus 1.54 g KMnO₄ in 25 ml H₂O sowie 0.6 g Benzyltriethylammoniumchlorid versetzt. Nach 4 h Rühren bei RT wurde die organische Phase abgetrennt, zweimal mit H₂O gewaschen und nach Trocknung über Na₂SO₄ eingedampft. Der Rückstand wurde durch Säulenchromatographie an Kieselgel 60 mit Dichlormethan/Methanol 9/1 als Eluens gereinigt und aus Ether kristallisiert. Ausb. 0.77 g (53 % d.Th.), Schmp. 190-193°C.

### Beispiel 5

### 9b-Phenyl-2,3-dihydrothiazolo-[2,3-a]isoindol-5(9bH)-on-1-oxid

0.5 g 9b-Phenyl-2,3-dihydrothiazolo-[2,3-a]isoindol-5(9bH)-on wurden in 5 ml Eisessig gelöst und nach Zugabe von 0.2 ml 30proz. H₂O₂ 10 h bei RT gerührt. Nach der Hälfte der Zeit wurden weitere 0.2 ml 30proz. H₂O₂ zugegeben.

Nach der angegebenen Zeit wurde der Ansatz eingedampft und der Rückstand durch Säulenchromatographie an Kieselgel 60 mit Dichlormethan/Methanol 97/3 als Eluens gereinigt. Ausb. 145 mg (27 % d.Th.), Schmp. 174-177°C.

## Patentansprüche

1. Verwendung von Verbindungen der allgemeinen Formel I 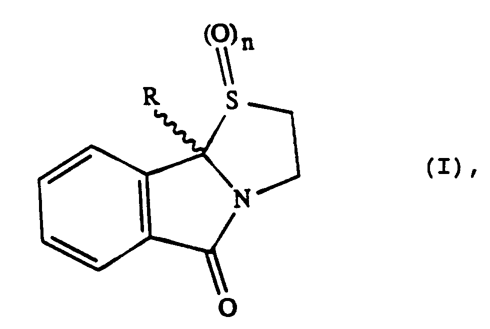 zur Herstellung von Arzneimitteln zur Behandlung von viralen oder retroviralen Infektionen, wobei
R ein Wasserstoffatom oder einen geradkettigen oder verzweigten, gesättigten oder ungesättigten aliphatischen Rest mit 1-7 Kohlenstoffatomen, der gegebenenfalls durch Phenyl substituiert sein kann, oder einen Phenylring, der gegebenenfalls ein- oder mehrfach durch C₁-C₄-Alkyl, C₁-C₄-Alkoxy, Hydroxy, Trifluormethyl, C₁-C₄-Alkylsulfonyl oder Halogen, wie Fluor, Chlor oder Brom, substituiert sein kann, bedeutet,
n die Zahlen 0, 1 oder 2 bedeutet,
sowie deren pharmakologisch verträgliche Salze oder Tautomere.

2. Verwendung von Verbindungen der Formel I gemäß Anspruch 1, wobei R ein Wasserstoffatom oder einen gesättigten oder ungesättigten, geradkettigen oder verzweigten aliphatischen Rest mit 1-7 Kohlenstoffatomen bedeutet.

3. Verwendung von Verbindungen der Formel I gemäß Anspruch 1, wobei R einen C₁-C₇-Alkylrest bedeutet, der durch eine Phenylgruppe substituiert sein kann.

4. Verwendung von Verbindungen der Formel I gemäß Anspruch 1, wobei R eine Phenylgruppe bedeutet, die unsubstituiert oder ein- bis dreifach substituiert ist durch C₁-C₄-Alkyl, Halogen, C₁-C₄-Alkoxy oder Hydroxy.

5. Verwendung von Verbindungen der Formel I gemäß Anspruch 1 ausgewählt aus der Gruppe der folgenden Verbindungen:
9b-Phenyl-2,3-dihydrothiazolo-[2,3-a]isoindol-5(9bH)-on
9b-(4-Methylphenyl)-2,3-dihydrothiazolo-[2,3-a]isoindol-5(9bH)-on
9b-(3,4-Dimethylphenyl)-2,3-dihydrothiazolo-[2,3-a]isoindol-5(9bH)-on
9b-(3-Methylphenyl)-2,3-dihydrothiazolo-[2,3-a]isoindol-5(9bH)-on
9b-(3-Chlorphenyl)-2,3-dihydrothiazolo-[2,3-a]isoindol-5(9bH)-on
9b-(3,5-Dichlorphenyl)-2,3dihydrothiazolo-[2,3-a]isoindol-5(9bH)-on
9b-(4-Chlorphenyl)-2,3-dihydrothiazolo-[2,3-a]isoindol-5(9bH)-on

6. Verwendung von Verbindungen der Formel I gemäß einem der Ansprüche 1-5 zur Herstellung von Arzneimitteln zur Behandlung von AIDS, ARC oder deren Vorstadien.

## Claims

1. Use of compounds of the general formula I 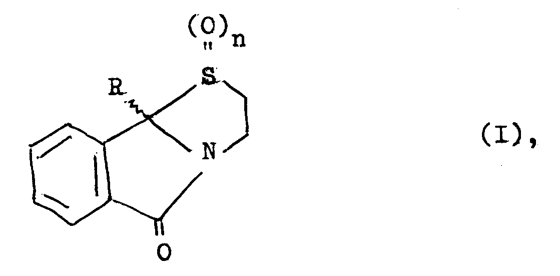 for the preparation of medicaments for the treatment of viral or retroviral infections, whereby R signifies a hydrogen atom or a straight-chained or branched, saturated or unsaturated aliphatic radical with 1 - 7 carbon atoms, which can possibly be substituted by phenyl, or a phenyl ring which can possibly be substituted one or more times by C₁-C₄-elkyl, C₁-C₄-alkoxy, hydroxyl, trifluoromethyl, C₁-C₄-alkyl-sulphonyl or halogen, such as fluorine, chlorine or bromine, n signifies the numbers 0, 1 or 2, as well as of their pharmacologically compatible salts or tautomers.

2. Use of compounds of the formula I according to claim 1, whereby R signifies a hydrogen atom or a saturated or unsaturated, straight-chained or branched aliphatic radical with 1 - 7 carbon atoms.

3. Use of compounds of the formula I according to claim 1, whereby R signifies a C₁-C₇-alkyl radical which can be substituted by a phenyl group.

4. Use of compounds of the formula I according to claim 1, whereby R signifies a phenyl group which is unsubstituted or is substituted one to three times by C₁-C₄-alkyl, halogen, C₁-C₄-alkoxy or hydroxyl.

5. Use of compounds of the formula I according to claim 1 selected from the group of the following compounds:
9b-phenyl-2,3-dihydrothiazolo[2,3-a]isoindol-5(9bH)-one
9b-(4-methylphenyl)-2,3-dihydrothiazolo[2,3-a]-isoindol-5(9bH)-one
9b-(3,4-dimethylphenyl)-2,3-dihydrothiazolo-[2,3-a]-isoindol-5(9bH)-one
9b-(3-methylphenyl)-2,3-dihydrothiazolo[2,3-a]-isoindol-5(9bH)-one
9b-(3-chloropnenyl)-2,3-dihydrothiazolo[2,3-a]-isoindol-5(9bH)-one
9b-(3,5-dichlorophenyl)-2,3-dihydrothiazolo[2,3-a]-isoindol-5(9bH)-one
9b-(4-chlorophenyl)-2,3-dihydrothiazolo[2,3-a]-isoindol-5(9bH)-one

6. Use of compounds of the formula I according to one of claims 1 - 5 for the preparation of medicaments for the treatment of AIDS, ARC or its preliminary stages.

## Revendications

1. Utilisation de composés de formule générale I 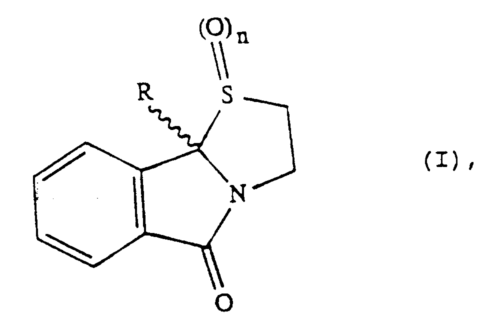 pour la préparation de médicaments destinés au traitement d'infections provoquées par des virus ou des rétrovirus, dans laquelle
R représente un atome d'hydrogène ou un reste aliphatique saturé ou insaturé, à chaîne linéaire ou ramifiée, ayant 1-7 atomes de carbone, qui peut être éventuellement substitué par un groupe phényle, ou un noyau phényle qui peut être éventuellement substitué une ou plusieurs fois par un groupe alkyle en C₁-C₄, alcoxy en C₁-C₄, hydroxy, trifluorométhyle, alkylsulfonyle en C1 -C₄, ou par un atome d'halogène, tel que le fluor, le chlore ou le brome,
n représente un nombre qui vaut 0, 1 ou 2,
ainsi que leurs sels pharmacologiquement acceptables ou leurs tautomères.

2. Utilisation de composés de formule I selon la revendication 1, dans laquelle R représente un atome d'hydrogène ou un reste aliphatique saturé ou insaturé, à chaîne linéaire ou ramifiée, ayant 1-7 atomes de carbone.

3. Utilisation de composés de formule I selon la revendication 1, dans laquelle R représente un groupe alkyle en C₁-C₇, qui peut être substitué par un groupe phényle.

4. Utilisation de composés de formule I selon la revendication 1, dans laquelle R représente un groupe phényle, qui n'est pas substitué ou qui est substitué une à trois fois par un groupe alkyle en C₁-C₄, un atome d'halogène, un groupe alcoxy en C₁ -C₄ ou hydroxy.

5. Utilisation de composés de formule I selon la revendication 1, choisis dans le groupe constitué par les composés suivants :
9b-phényl-2,3-dihydrothiazolo-[2,3-a]isoindol-5(9bH)-one
9b-(4-méthylphényl)-2,3-dihydrothiazolo-[2,3-a]isoindol-5(9bH)-one
9b-(3,4-diméthyphényl)-2,3-dihydrothiazolo-[2,3-a]isoindol-5(9bH)-one
9b-(3-méthylphényl)-2,3-dihydrothiazolo-[2,3-a]isoindol-5(9bH)-one
9b-(3-chlorophényl)-2,3-dihydrothiàzolo-[2,3-a]isoindol-5(9bH)-one
9b-(3,5-dichlorophényl)-2,3-dihydrothiazolo-[2,3-a]isoindol-5(9bH)-one
9b-(4-chlorophényl)-2,3-dihydrothiazolo-[2,3-a]isoindol-5(9bH)-one

6. Utilisation de composés de formule I, selon l'une quelconque des revendications 1-5, pour la préparation de médicaments destinés au traitement du SIDA, du syndrome associé au SIDA ou de leurs stades précurseurs.
